# EUROPEAN PATENT SPECIFICATION

(11) **EP 1 024 807 B1**
(45) Date of publication and mention of the grant of the patent: **16.04.2003**
(21) Application number: 97935773.8
(22) Date of filing: 08.08.1997
(51) Int. Cl.: A61K 31/40, A61P 13/12

(54) **USE OF THE COMPOUND 2-METHOXYPHENYL-1-METHYL-5P-METHYLBENZOYL-PYRROL-2-ACETAMIDO ACETATE FOR THE PRODUCTION OF AN ANTI-INFLAMMATORY DRUG WITH PREVENTION OF GASTRIC HYPERSECRETION AND RENAL IMPAIRMENT**
VERWENDUNG VON 2-METHOXYPHENYL-1-METHYL-5P-METHYL-BENZOYLPYRROL-2-ACETAMIDO-ACETAT ZUR HERSTELLUNG EINES ANTIENTZÜNDUNGSMITTELS MIT VORBEUGUNG VON MAGENHYPERSEKRETION UND NIERENFUNKTIONSSTÖRUNG
UTILISATION DU COMPOSE 2-METHOXYPHENYL-1-METHYL-5P-METHYLBENZOYL-PYRROL-2-ACETAMIDO-ACETATE POUR LA PRODUCTION D'UN MEDICAMENT ANTI-INFLAMMATOIRE AVEC PREVENTION DE L'HYPERSECRETION GASTRIQUE ET DE L'INSUFFISANCE RENALE

(43) Date of publication of application: 09.08.2000
(60) Divisional application: 02078447.6
(73) Proprietor: MEDOSAN Ricerca S.r.l., 00040 Cecchina RM (IT); Sigma Tau Industrie Farmaceutiche Riunite S.p.A., 00144 Roma (IT)
(72) Inventor: ANZALONE, Sergio, I-00183 Roma (IT)
(74) Representative: Bazzichelli, Alfredo
(86) International application number: IT9700209
(87) International publication number: WO99007363

(56) References cited:
- EP-A- 0 755 679
- US-A- 4 578 481
- DIALOG FILE SUPPLIER: FILE 129: PHIND; AN=560550; SCRIP 2290, 5 December 1997, page 9 XP002061890
- TUBARO E ET AL: "STUDIES ON THE GASTRIC TOLERABILITY OF THE NEW NON-STEROIDAL ANTI-INFLAMMATORY DRUG AMTOLMETIN GUACYL" ARZNEIMITTEL FORSCHUNG. DRUG RESEARCH, vol. 45, no. 12, December 1995, pages 1298-1302, XP000611666
- M. DELFINO ET AL.: "EVALUATION OF THE THERAPEUTIC ACTIVITY AND GASTROLESIVE EFFECTS OF A NEW NSAID VS PLACEBO IN PATIENTS WITH OSTEOARTICULAR DISEASES" CLIN TER., vol. 147, no. 3, 1996, pages 113-116, XP002061891
- M. LINGETTI ET AL.: "AN EVALUATION OF THE THERAPEUTIC ACTIVITY OF ST-679 IN PATIENTS WITH OSTEOARTHRITIS AT DIFFERENT SITES" CLIN. TER., vol. 142, no. 1 PT 2, 1993, pages 29-40, XP002061892
- G. DONATI ET AL.: "A CLINICAL STUDY INTENDED TO ESTABLISH THE OPTIMUM DOSAGE OF ST-679 IN RHEUMATIC DISORDERS" CLIN TER., vol. 142, no. 1, 1993, pages 19-28, XP002061893
- CARUSO A ET AL: "PHARMACOLOGICAL PROPERTIES AND TOXICOLOGY OF MED-15, A PRODRUG OF TOLMETIN" DRUGS UNDER EXPERIMENTAL AND CLINICAL RESEARCH, vol. 18, no. 11/12, 1992, pages 481-485, XP000610913

## Description

The present invention relates to the use of the compound 2-methoxyphenyl-1-methyl-5p-methylbenzoylpyrrol-2-acetamido acetate (known also as amtolmetin guacyl and indicated in the present description also as MED 15) for the production of a NSAID for treating an inflammatory pathological condition with effect of prevention of NSAID-related renal impairment due to vasoconstriction.

### Background Art

Non-steroidal anti-inflammatory drugs (NSAIDs) have been used for many years in therapy. It is also well known that NSAIDs produce lesions to gastrointestinal apparatus depending on the length of the treatment and on the type of drug. This problem has a dramatic importance in cases where the therapy must be protracted for a long time. An example is rheumatoid arthritis in old people, where a chronic treatment is needed to keep under control the inflammatory state and the pain and make acceptable the quality of life.

At present there is a pharmacological dogma establishing a mandatory connection between anti-inflammatory effect and gastric lesions. This dogma has been recently shaken by the availability of newly synthesised anti-inflammatory drugs showing gastrolesive effects lower than those of old ones. Notwithstanding that equiactive doses of drug could produce lesions of different seriousness, it was settled that an anti-inflammatory drug showed, in any case, a gastrolesive effect.

It is also well known, and this has a pharmacological relevance too, that the administration of NSAIDs reflects negatively on renal function, in particular by provoking a remarkable decrease in diuresis. This effect is due to prostaglandin blockade; in fact, prostaglandin physiologically dilate the renal vascular system. The blocking effects of traditional NSAIDs lead to vasoconstriction and to the consequent inhibition of diuresis. From this fact it followed that it could not be expected that these drugs known in the art could be administered without negatively affecting the renal function but on the contrary increasing the diuresis.

In view of the above, it could not be expected that a NSAID could maintain a high anti-inflammatory activity without negatively affect the renal function.

It is also well known that in view of their side-effects on gastric mucosa, NSAIDs are invariably administered after meals or, in general, when the stomach is not empty. The generality of this pharmacological principle finds a practical confirmation in the recommendations found in the packagings of the drugs in commerce. Basically the idea is that the effects of the hypersecretion of hydrochloric acid provoked by the administration of NSAIDs may be, at least partially, counteracted by the presence of food.

It has been now surprisingly found that the compound 2-methoxyphenyl-1-methyl-5p-methylbenzoyl-pyrrol-2-acetamido acetate, which is an effective anti-inflammatory non-steroidal drug as already known in the art, does not affect the renal function, but, on the contrary, increases the diuresis. This increase in diuresis is linked to its mechanism of action; in fact, the overflow of neuropeptides (in particular CGRP) produced in the stomach by MED 15 and flowing from the stomach into the bloodstream, produces renal capillary vasodilatation, with reversal of its effect on prostaglandin.

Additionally, and more than unexpectedly for a NSAID, it has been found that amtolmetin guacyl, in order to show its efficacy (good renal tolerability together with an anti-inflammatory effect unchanged), has to be administered on empty stomach in view of the peculiarity of its action mechanism.

The compound 2-methoxyphenyl-1-methyl-5p-methylbenzoyl-pyrrol-2-acetamido acetate is already known in the art and is for instance disclosed in Italian patent application no. 47881A/82 and in US patent no. 4,578,481 issued on March 25, 1986.

Both the documents of the state of the art disclose that the above compound shows anti-inflammatory, analgesic, antipyretic, antitussive and antisecretive (on the mucus of the respiratory airway) properties. No mention, either direct or indirect, is made about a possible antisecretive effect on the gastric secretion in mammals.

European patent application No. 96830388.3 discloses the use of amtolmetin guacyl for the production of a NSAID with simultaneous antisecretory activity on gastric secretion. Nothing is said, either directly or indirectly, in this document about a possible effect on renal vasoconstriction or attainment of maximum activity of amtolmetin guacyl.

It is therefore the subject matter of the present invention the use of the compound 2-methoxyphenyl-1-methyl-5p-methylbenzoyl-pyrrol-2-acetamido acetate for the production of a NSAID for treating NSAID-related renal impairment due to vasoconstriction. A further subject matter is the attaining of the maximum activity of this compound.

### BRIEF DESCRIPTION OF THE DRAWINGS

With the present description are enclosed eighteen sheets of drawings in which:

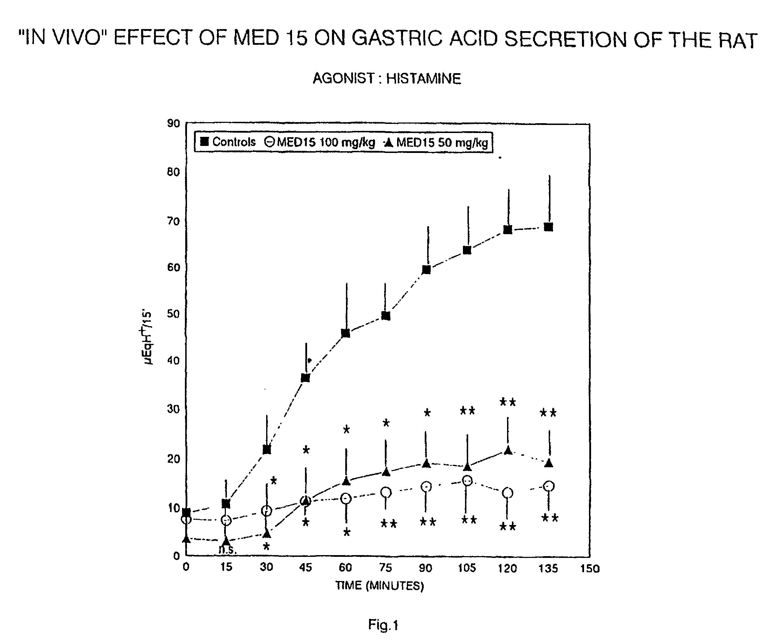

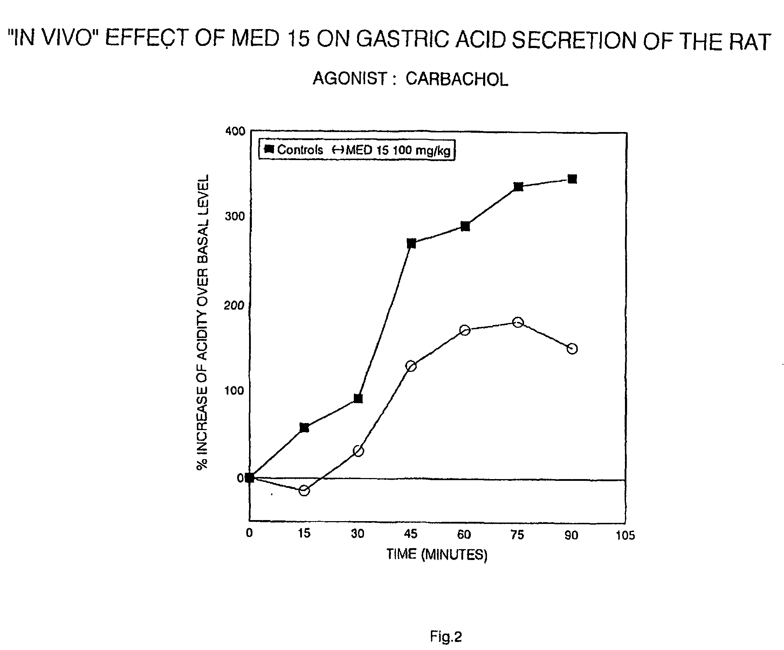

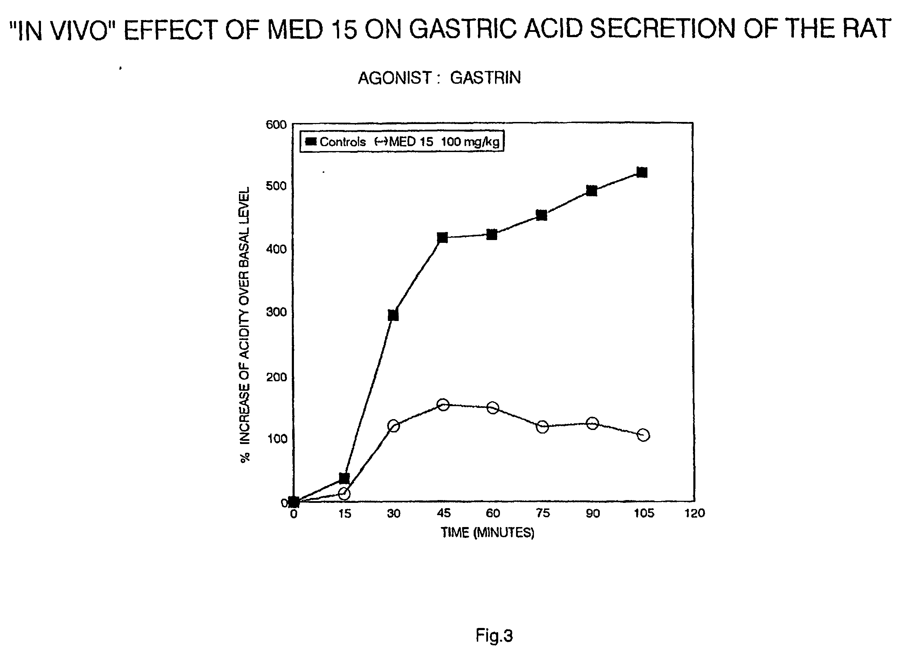

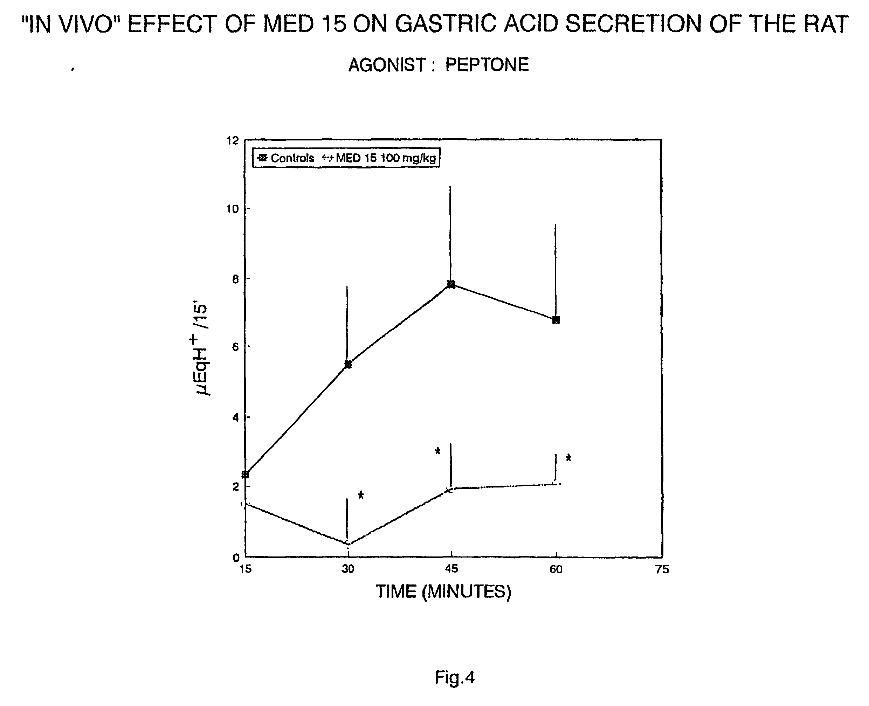

figs. 1 ,2, 3 and 4 show the "in vivo" effect of amtolmetin guacyl on gastric acid secretion of the rat, using as an agonist histamine, carbachol, gastrin and peptone, respectively;
fig. 5 shows the effect of amtolmetin guacyl on the H₂-receptors of the guinea pig isolated atrium; the stimulation was produced by histamine;
fig. 6 shows the effect of amtolmetin guacyl on gastric bicarbonate secretion in the rat, using histamine as a stimulating agent;
figs. 7 and 8 show the antispasmodic effect of amtolmetin guacyl on guinea pig isolated ileum, using as a stimulating agent histamine and acetylcholine (cumulative curves);
fig. 9 shows the antispasmodic effect of amtolmetin guacyl on guinea pig isolated ileum, using as a stimulating agent serotonin;
figs. 10 and 11 show the effect of amtolmetin guacyl on gastric motility in the rat, using respectively increasing doses of acetylcholine and serotonin, while fig. 12 the dose-effect relationship of amtolmetin guacyl on gastric motility in the rat;
fig. 13 shows the effect of amtolmetin guacyl and of diclofenac on rat diuresis;
fig. 14 shows the effect of amtolmetin guacyl on peptone-stimulated gastric acid secretion in the rat following treatment with CGRP₈₋₃₇;
fig. 15 shows a comparison among the molecular structures of capsaicin, nonanoyl vanillylamide and amtolmetin guacyl;
fig. 16 shows the effects of amtolmetin guacyl on peptone-stimulated gastric acid secretion in the rat following treatment with capsazepine;
fig. 17 shows the interference of diphenhydramine on gastroprotective action of amtolmetin guacyl in the rat; and
fig. 18 shows interference of diphenhydramine on the effect of amtolmetin guacyl on peptone-stimulated gastric acid secretion in the rat.

### CHEMISTRY

The molecule of amtolmetin guacyl was synthesised using modern chemical technology now known as "Combinatorial Chemistry". This neologism was coined in the United States as recently as 1993, but the technique was known years before. This new synthetic chemistry technique consists in combining molecular species bioactive in themselves, to obtain molecules which possess pharmacological properties totally different from those of the single starting components. Traditionally, a staring molecule was manipulated through the substitution of a number of radicals, now the procedure is less random and starts from molecular moieties which are pharmacologically active per se (Combinatorial Chemistry, Editorial Feb. 12, 1996, C&EN). The amtolmetin guacyl molecule was born this way, producing a drug with completely new characteristics, totally different from the bioactivity of the various moieties.

### 2) Pharmaco-toxicological profile

### Anti-inflammatory activity

### Amtolmetin guacyl as an anti-inflammatory agent as confirmed by the pharmacological studies

Moreover, as with all the NSAIDs currently aveilable, it inhibits gastric PGE₂ in the rat, albeit to a lesser degree than ASA (Table 7).

**Table 7.**

| Effect of MED15 and of ASA* on gastric PGE₂ in the rat | | | |
|---|---|---|---|
| Groups | PGE₂ (ngtg^{Δ} tissue)±S.E. | %inhibition | P |
| Controls | 108.7±23.9 | | |
| MED15 | 32.7±8.5 | 69.9 | 0.022 |
| ASA | 14.4±4.5 | 86.7 | 0.003 |

| | | | |
|---|---|---|---|
| (*)= acetyl salicylic acid | | | |
| (Δ)= nanograms for gram of tissue | | | |

In order to have a full evaluation of the anti-inflammatory activity of the amtolmetin guacyl, a number of experimental tests have been carried out to duplicate the results already available to the Applicant. The tests have been carried out by the William Harvey Institute as follows.

### Carrageenan pleurisy

### METHODS

### Dosing

Compounds were administered orally 1 hour prior to and 1 hour post pleurisy induction in a volume of 0.5 ml 1% gum tragacanth. Indomethacin was used at 3 mg/kg. MED 15 was used at 25, 50 and 100 mg/kg.

### Preparation of carrageenan

A 1% solution of carrageenan in sterile saline was prepared. The solution was placed in an incubator at 37°C for half an hour to allow complete hydration of the carrageenan. The solution was then mixed to homogeneity.

### Induction

A 21Gx40mm syringe needle was reduced in length to 5mm and fitted to a 1ml syringe containing the irritant. Rats were lightly anaesthetised with halothane. Each animal was placed on its left side. The skin over the thorax was lifted with and a 1cm incision made through the skin with a pair of scissors. The wound was opened to expose the underlying muscle. Using a scalpel, a 2-3mm incision was made into the muscle of the 5th-6th intercostal space. A volume of 0.15ml of carrageenan solution was then injected into the pleural cavity. The wound was closed with a 11mm Michel clip and the animal allowed to recover from the anaesthetic. Groups of animals were killed 4 hours after pleurisy induction.

### Collection of pleural exudate

Lavage solution: One part of 3.15% trisodium citrate solution, mixed with nine parts of Hank's balanced salt solution.

Rats were killed by over exposure to carbon dioxide. For each rat the skin over the thorax was moistened with IMS alcohol. The skin over the sternum was then lifted and a 5cm cut made, to expose the musculature of the upper abdomen and thoracic cavity. The muscle overlying the xiphisternum was lifted with tissue forceps and a 1cm cut made through the muscle to reveal the cartilage. This was then lifted and the diaphragm perforated just beneath the cartilage. Two cuts were then made through the rib cage either side of the cartilage to form a flap which could be folded back to expose the thoracic cavity.

Using a syringe, 1ml of lavage fluid was introduced into the thoracic cavity. The fluid was aspirated 2-3 times with a 3ml pastette to wash the cavity, before being collected into separate 10ml conical-based plastic test-tubes. Any blood contaminated fluids were rejected.

### Quantification of exudates

The mass of each tube with fluid was determined. By subtracting the mass of an empty tube from these values, and assuming a density for the fluid of 1g/cm³, the fluid volume was quantified.

### Statistical analysis

Results were analysed by ANOVA followed by Bonferroni T-test. Values of p<0.05 were considered significant.

### RESULTS

Results for exudated volume are shown in Table 8. Dosing was one hour prior to and one hour post pleurisy induction and inflammation was assessed at 4 hours.

### Exudate volume

Exudate volume for gum tragacanth control was 0.94±0.04ml. Indomethacin at 3mg/kg caused a 50% reduction in exudate volume (p<0.01) compared to gum tragacanth control. MED 15 at all concentrations tended to decrease exudate volume by 11%, 21% and 44% at 25, 50 and 100mg/kg respectively; with the highest dose giving a statistically significant reduction (p<0.05) compared to gum tragacanth control.

**Table 8**

| Carrageenan pleurisy (4 hour) | | | | | |
|---|---|---|---|---|---|
| | Control (gum tragacanth) (n=10) | Indomethacin 3mg/kg (n=11) | MED 15 25mg/kg (n=9) | MED 15 50mg/kg (n=10) | MED 15 100mg/kg (n=9) |
| Exudate volume (ml) | 0.94±0.04 | 0.47±0.11 | 0.84+0.09 | 0.74±0.06 | 0.53±0.05 |
| | | P<0.01 | p=NS | p=NS | P<0.05 |

### Adjuvant arthritis

### METHODS

### Dosing

The drugs were administered daily by oral gavage for 4 days (day 0-3); piroxicam and MED 15 were dissolved in 5% Gum arabic + 0.01% Tween 80, and administered in a volume of 0.5ml. Piroxicam was administered as 3mg/kg and MED 15 was administered as 25, 50 and 100mg/kg doses.
Table 8 bis illustrating dosage regimes.
L=left stifle joint, R=Right stifle joint.

| | Arthritis | induction | Daily |
|---|---|---|---|
| Treatment | 25µl | 200 µg | 0.5ml |
| | Saline | M.tb. | Drugs |
| Non-arthritic saline + vehicle | L & R | - | Orally |
| Arthritis + vehicle | R | L | Orally |
| Piroxicam 3mg/kg | R | L | Orally |
| MED 15 25mg/kg | R | L | Orally |
| MED 15 50mg/kg | R | L | Orally |
| MED 15 100mg/kg | R | L | Orally |

### Induction of arthritis

Under halothane anaesthesia, rats received 200µg of heat-killed finely ground *Mycobactyerium tubercolosis* in 25µl (80mg/10ml sterile saline) injected intra-articulary into the left stifle joint. The contralateral joint received 25µl 0.9% sterile saline. Control animals
received 25µl of 0.9% sterile saline in both knees. SGE 50µl microsyringes with a volume lock calibrated at 25µl were used with 30G stainless steel needles. Solutions were prepared sterile, and aseptic technique was used throughout, with sterilisation of syringes between groups.

### Joint inflammation

The rats were killed by carbon dioxide suffocation. The body weight was noted and the skin overlying the stifle joints was divided and the joint diameters assessed in mm using Mitutoyo vernier callipers (530-312, 0.02mm).

### Patellar bone erosion

The patellae were dissected, weighed (Sartorius, 0.01mg) and then immersed in 1ml digestion buffer (see below) to remove soft tissue.

Digestive buffer was prepared fresh to the following formula; disodium hydrogen orthophosphate 20mM, EDTA 1mM and diothiothreitol at 2mM. Papain (EC 3.4.22.2) was added at a concentration of 6U/ml. Each patella was digested in 1ml of digestive buffer at 56°C for 4 hours. The residual paterllar bones were dried (100°C 24 hours) and weighed (Sartorius 0.01mg).

### Calculations and statistical analysis

Results are presented as follows: left and right parameter alone, the difference between arthritic and contralateral control joint (left-right); and % difference between arthritic and control joints ([left-right]/right]X100).

### Units

| | |
|---|---|
| Joint diameter | mm |
| Patellar bone mass | mg |

Calculations were carried out using the spreadsheet program Lotus 123. Statistical comparisons were made between the absolute differences using INSTAT Mann Whitney unpaired two-tailed statistical test.

Table 9. The effects of daily oral dosing of piroxicam or MED 15 in rat knee joint diameters.

| Treatment | n | Joint diameter (mm) | | Swelling Diameter difference | % Change |
|---|---|---|---|---|---|
| | | Left | Right | (L-R) | |
| Non-arthritic vehicle | 10 | 8.53 | 8.63 | -0.10 | -1.17 |
| controls | | ±0.08 | ±0.08 | ±0.04 | ±0.51 |
| Arthritic vehicle | 10 | 12.22 | 8.69 | 3.53 | 40.66 |
| controls | | ±0.18 | ±0.11 | ±0.15 | ±1.78 |
| Piroxicam 3mg/kg | 10 | 10.41 | 8.74 | 1.67 | 19.15 |
| | | ±0.09 | ±0.05 | ±0.10 | ±1.17 |
| MED 15 25mg/kg | 10 | 11.57 | 9.35 | 2.22 | 24.04 |
| | | ±0.20 | ±0.21 | ±0.14 | ±1.78 |
| MED 15 50mg/kg | 10 | 10.95 | 8.88 | 2.06 | 23.36 |
| | | ±0.14 | ±0.15 | ±0.16 | ±1.98 |
| MED 15 100mg/kg | 9 | 10.26 | 8.88 | 1.38 | 15.71 |
| | | ±0.10 | ±0.15 | ±0.14 | ±1.73 |

### Patellar bone mass

Table 10 shows the effects of drug treatments on patellar bone mass. There was a large reduction in bone mass in the arthritic (p<0.0001) groups compared to the non-arthritic group reflecting erosion. Although there was no statistically significant difference in the loss of bone mass of the arthritic groups the piroxicam treated group showed the greatest protection against bone loss. MED 15 displayed a dose dependent protective effect which was not significantly different to piroxicam at a dose of 100mg/kg.

Table 10 The effects of daily oral dosing of piroxicam or MED 15 on rat patellar bone mass.

| Treatment | n | Bone mass (mg) | | Bone loss Left-Right (mg) | % Change |
|---|---|---|---|---|---|
| | | Left | Right | | |
| Non-arthritic vehicle | 10 | 3.59 | 3.73 | -0.09 | -3.39 |
| controls | | ±0.16 | ±0.11 | ±0.13 | ±2.90 |
| Arthritic vehicle | 10 | 2.03 | 3.96 | -1.93 | -48.06 |
| controls | | ±0.31 | ±0.15 | ±0.34 | ±7.97 |
| Piroxicam 3mg/kg | 10 | 3.07 | 4.21 | -1.31 | -27.74 |
| | | ±0.13 | ±0.09 | ±0.11 | ±2.26 |
| MED 15 25mg/kg | 10 | 2.33 | 4.27 | -1.94 | 45.45 |
| | | ±0.18 | ±0.0.8 | ±0.18 | ±4.13 |
| MED 15 50mg/kg | 10 | 2.49 | 4.18 | -1.69 | 40.55 |
| | | ±0.14 | ±0.10 | ±0.12 | ±2.82 |
| MED 15 100mg/kg | 9 | 2.97 | 4.34 | -1.38 | 31.85 |
| | | ±0.12 | ±0.08 | ±0.08 | ±1.93 |

### Toxicological profile

From a toxicological viewpoint the drug offers an excellent torability profile. In fact, the drug safety trials demonstrated absence of effects on blood pressure and on the cardiovascular system (b.r. No. 4).

Acute toxicity studies (b.r. No. 5), indicated excellent drug tolerability. Studies carried out by the Hazleton laboratories (b.r. No. 6 and 7) in particular, demonstrated absence of gastric damage following chronic oral administration for 52 weeks in the rat and in the Cynomolgous monkey.

The fertility, embriotoxicity and teratogenicity studies in the rat and in the rabbit (b.r. No. 8, 9 and 10) demonstrated the safety product.

In the same way the Ames test (b.r. No. 11), forward mutation in Saccharomycetes pombe Pl (b.r. No. 12), mitotic conversion in Saccharomycetes D4 (b.r. No. 13), chromosomic aberrations in human lymphocites cultivated in vitro (b.r. No. 14), micronucleus test in rat bone marrow (b.r. No. 15) all demonstrated absence of amtolmetin guacyl-related damage.

Carcinogenesis studies in the rat administered by gavage with amtolmetin guacyl for a period covering the animal's life-span (b.r. No. 16) demonstrated the innocuity of the product.

### 3) Mechanism of action

Interest in the amtolmetin guacyl molecule arose following a thorough analysis of the results of 31 comparative and 6 non-comparative clinical trials involving 1596 patients. All of the studies revealed an extremely low incidence of adverse events involving the stomach (5% circa) which compared the product very favourably with the other NSAIDs, in which the incidence was in the region of 30% or more (as in the case of indomethacin) with frequent interruptions of treatment. This 5% was considered by us to be practically null in consideration of the fact that all of the patients reporting side effects frequently had a past history of concomitant disease and previous medical treatment; also, it cannot be excluded that antihistamine drugs were taken during the course of the experimentation which are known to interfere with the mechanisms of gastric protection of amtolmetin guacyl. Turthermore the physician, being aware of administering an NSAID, was expecting adverse events and could not be expected to be an impartial observer. The few cases of gastro-intestinal phenomena reported with amtolmetin guacyl were transitory and in no event accompanied by the presence of occult blood in the stools. Moreover, even in the presence of a reported side-effect it was almost never necessary to suspend treatment (0.4% of suspensions).

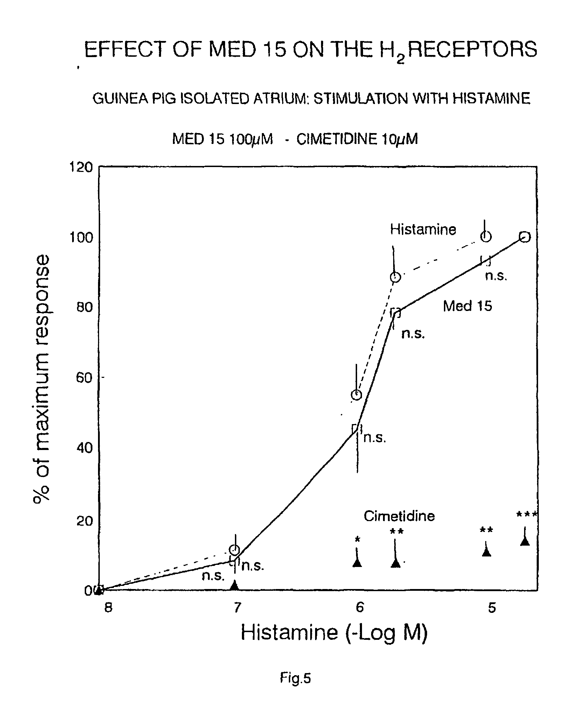

On the basis of the above series of observations, we decided to study the pharmacological behaviour of a number of widely used NSAIDs (including amtolmetin guacyl) towards the gastric mucosa in the rat, as previously described. Having univocally established the effect of amtolmetin guacyl on the reduction of gastric acid, the question of the mechanism of action of this effect arose. The first hypothesis was that we were deling with an anti-H₂ drug, given the fact that its potency could be compared with that of cimetidine. However, when using an organ in which only H₂ receptors were present (guinea-pig atrium) the drug did not interfere with the H₂ receptors (fig. 5).

On the other hand, we had previously noted differences between potentiometric determination of H⁺ and NaOH titration of the gastric effluate, in agreement with the substantial literature available on the subject, This fact was clearly in favour of a strong presence of bicarbonates (b.r. No. 27, 28).

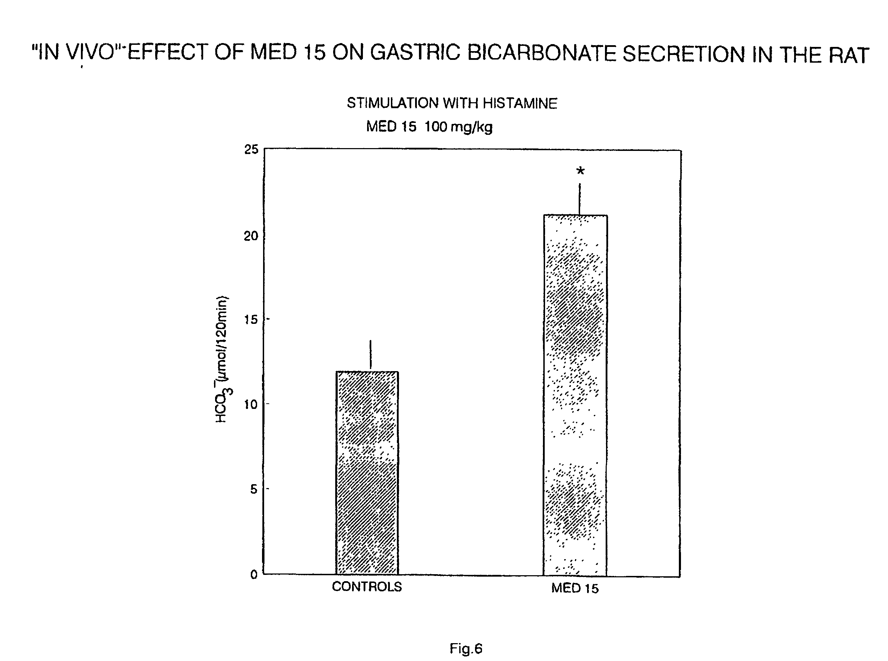

Experiments on bicarbonate secretion (under the same conditions as these of the acid secretion studies) following stimulation with histamine and treatment with amtolmeting guacyl, demonstrated an increase in bicarbonate production of 67% (fig. 6).

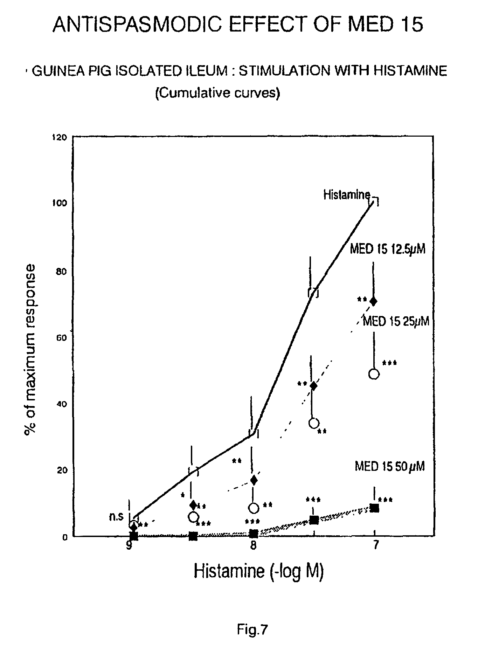

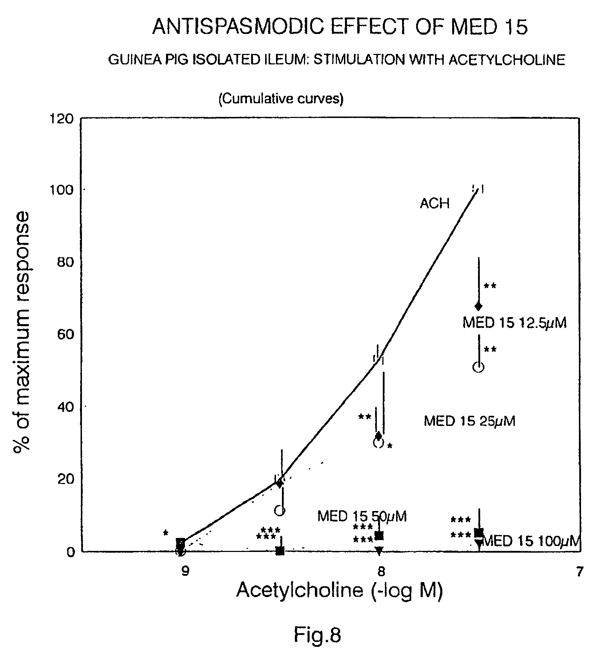

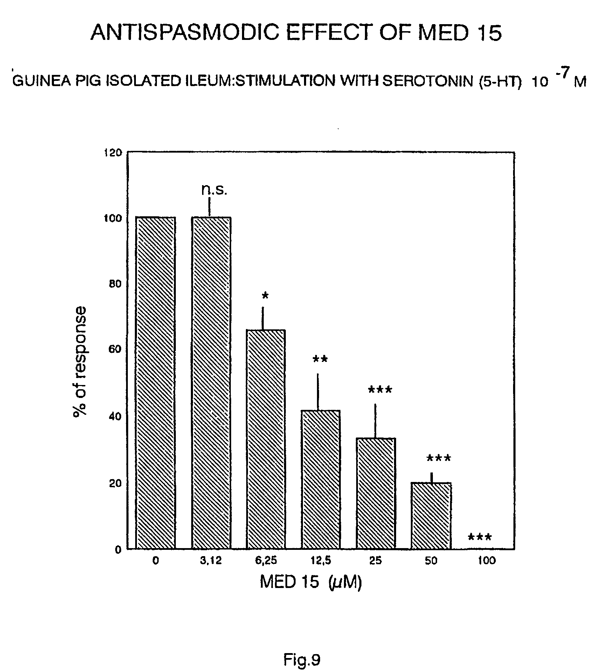

In parallel we also studied the possible effect on intestinal motility in the guinea-pig isolated ileum (b.r. No. 29) using various agonists (fig. 7, 8 and 9).

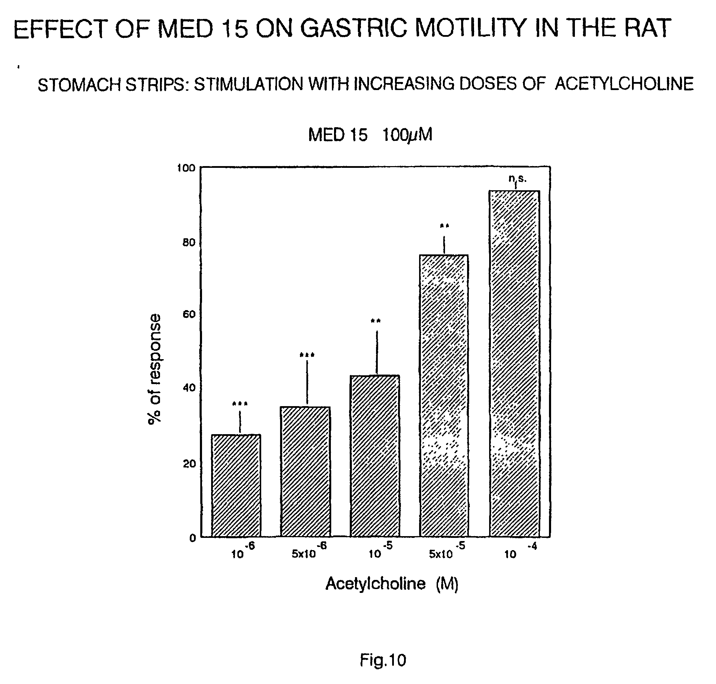

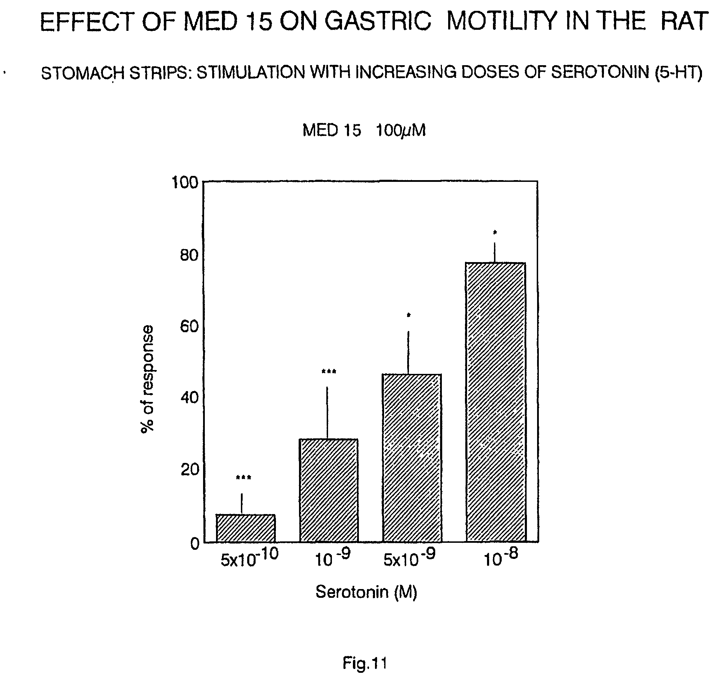

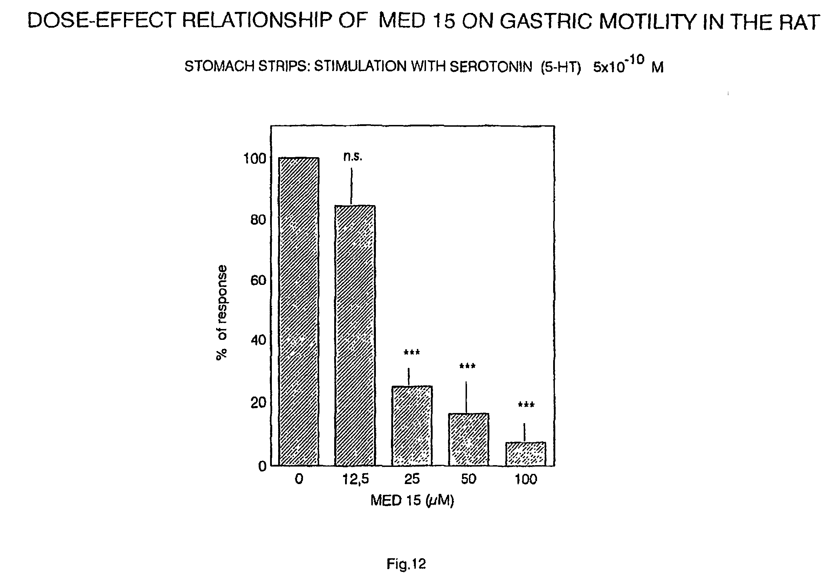

Having established the amtolmeting guacyl-related decrease in motility using all of the traditional agonists, we proceeded to verify whether this same effect was present at gastric level. Using acetilcholine and serotonin as agonists in the rat isolated stomach strips model (b.r. No. 30), we obtained clear evidence of down-regulation of motility also in this organ (fig. 10, 11 and 12).

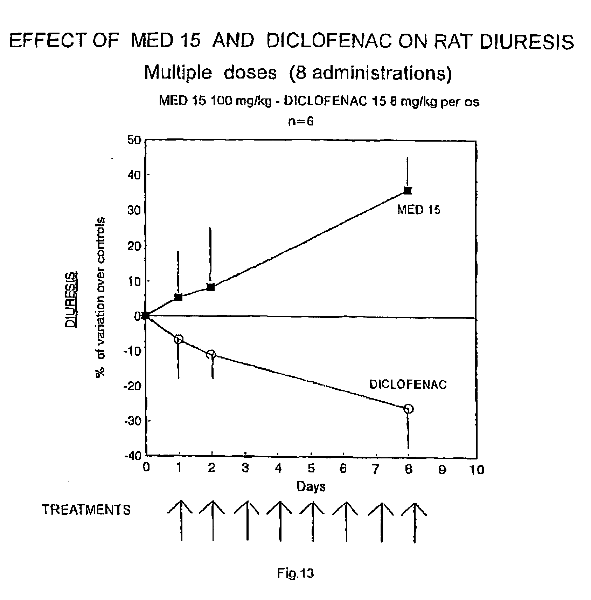

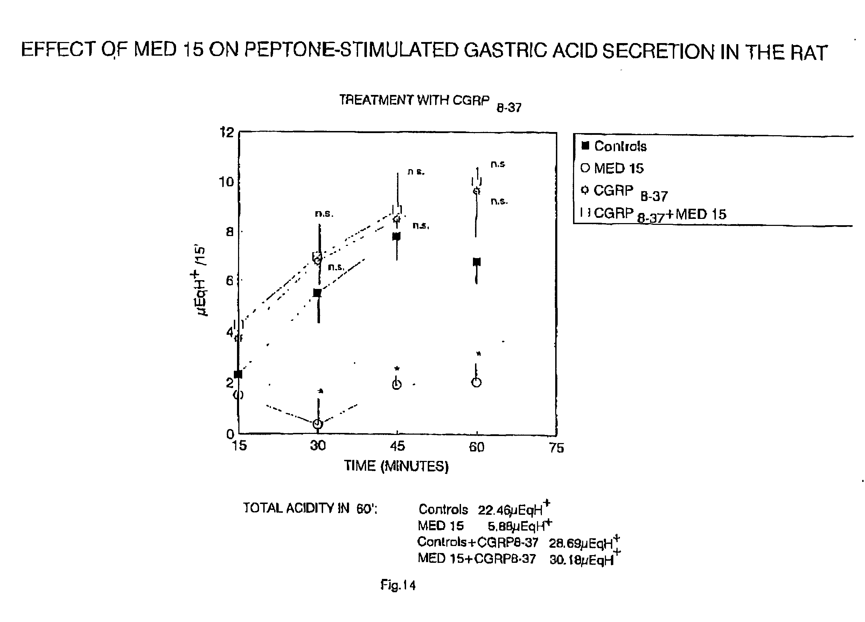

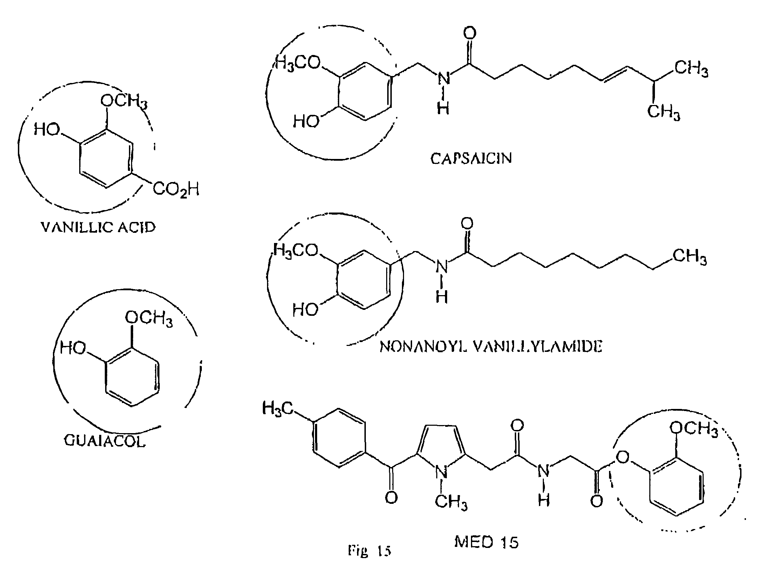

In the course of various studies conducted on animals to show the lack of renal impairment, a test was carried out to compare the diuresis in normally hydrated rats treated orally for 8 consecutive days with amtolmeting guacyl and diclofenac, which is also a NSAID. From the results, which are shown in figure 13, it is possible to observe a definite increase in diuresis in the animals which were treated with amtolmeting guacyl and a likewise clear decrease in the animals treated with diclofenac. It is to be stressed that the behaviour of diclofenac is completely in line with the behaviour of a NSAID as known in the art, that is to say to markedly reduce the diuresis, while that of amtolmeting guacyl is completely unforeseeable.

The results obtained led to the identification of a mechanism of action which would simultaneously account for:
1) - decrease of acidity
2) - increase of bicarbonates
3) - increase of gastric blood flow
4) - increase of diuresis
5) - decrease of motility.

At this stage CGRP (calcitonin gene related peptide) was considered a good candidate to match with all of the parameters to be considered. CGRP is a 37 aminoacid polipeptide which depresses gastric acidity (b.r. No.31, 32).

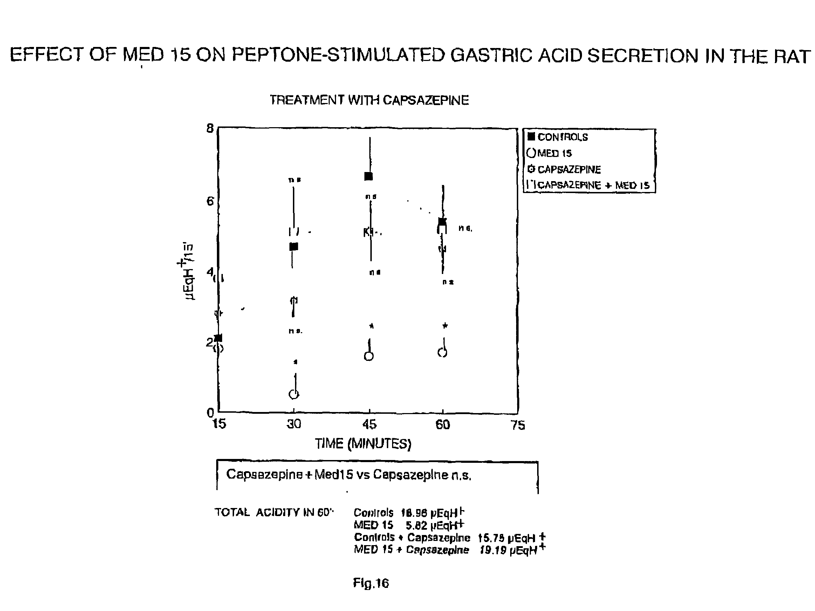

Experiments carried out using a specific antagonist of CGRP (CGRP₈₋₃₇) demonstrated that inhibition of this polipeptide leads to blockage of the amtolmetin guacyl - related activity on gastric secretion. This demonstrates thet CGRP actually is implicated in the mechanism of action of amtolmetin guacyl(fig. 14).

These results were confirmed by Huntingdon Life Sciences U.K.

CGRP production itself is known to be stimulated by the vanilloid (or capsaicin) sensitive receptors which simultaneously produce an increase in bicarbonate production (b.r. No. 33). A comparison between the capsaicin structures and amtolmetin guacyl shows that all of these structures contain a vanilloid radical in their molecules (fig. 15).

The hypothesis of the impact of amtolmetin guacyl on the vanilloid receptors was therefore strengthened and the experimental demonstration was obtained using capsazepine, a specific receptor antagonist of capsaicin (fig. 16). The introduction of a guaiacol moiety into the amtolmetin guacyl molecule led to the insertion of a vanilloid radical which confers gastroprotective properties to the molecule. Moreover, although capsaicin possesses revulsive and inhibitory properties, both nonivamide and amtolmetin guacyl are inhibited by the capsazepine antagonist, and do not determine any irritative phenomena (b.r. No. 34, 35, 36).

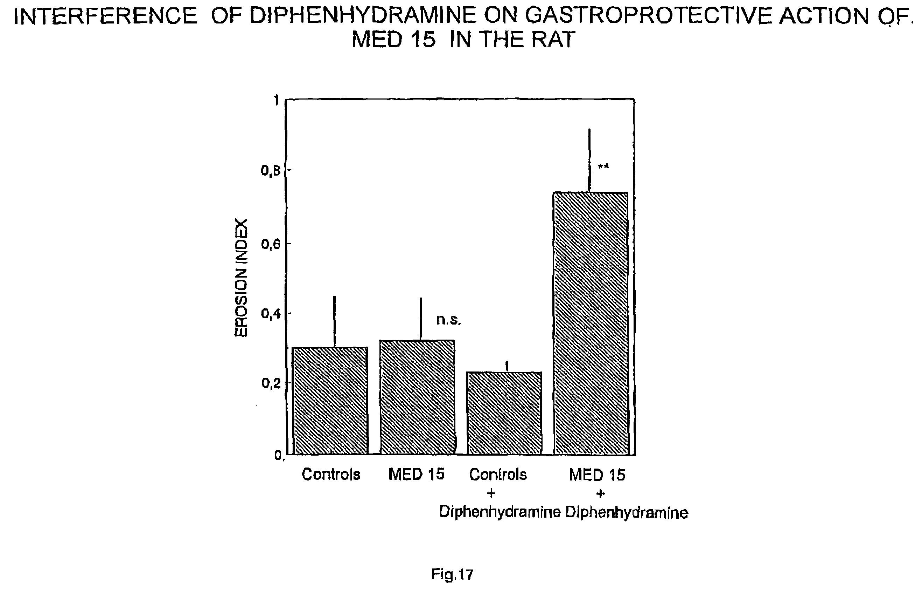

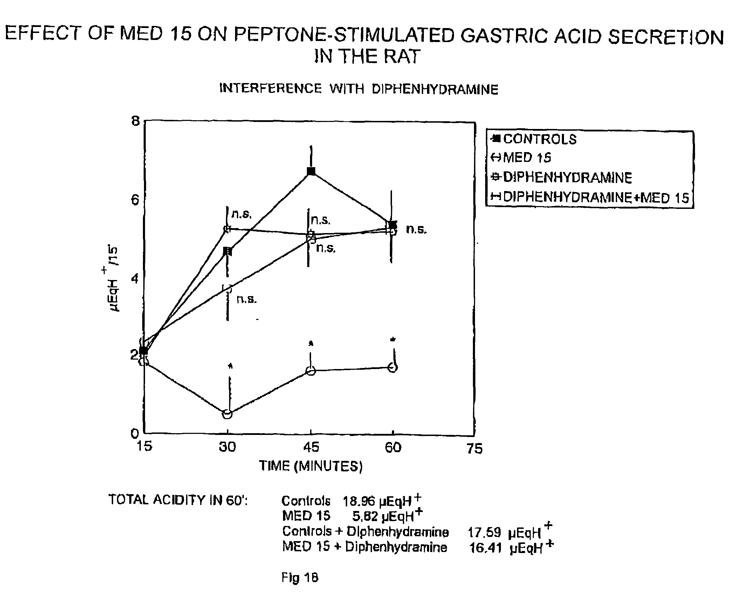

In the *in vivo* experimentation, evaluated as effect of the product both on gastric mucosal secretion and on the healthy condition of the mucosa itself, it was noted that in the presence of an inhibitor of H₁ receptors (therefore a typical antihistamine agent), the gastroprotective effect of amtolmetin guacyl drops out. This is a further demonstration of the impact of amtolmetin guacyl on the vanilloid receptors, due to the well known interference existing between these receptors and drugs such as diphenidramine (b.r. No. 37) and pyrilamine (b.r. No.38). All of this constitutes a precise forewarning of the association of amtolmetin guacyl with H₁ receptor inhibiting substances on pain of loss of the gastroprotective properties of the former (fig. 17 and 18).

The presence of the vanilloid radical, essential to the expression of the gastroprotective mechanism, is guaranteed by the prolonged persistence of high amounts of intact amtolmetin guacyl molecule in the gastric and intestinal walls (at least 2 hours after oral administration in the rat).

CGRP down-regulates acid secretion and also produces intense vasodilation of gastric mucosa and of the kidney, where it produces an increase in glomerular infiltration rate (which justifies the diuretic effect of amtolmetin guacyl)(b.r. No. 39). Also, the decrease in amtolmetin guacyl-determined motility which we observed may be attributed to increased CGRP, a notorious inhibitor of gastrointestinal motility (b.r. No. 40).

Recent studies indicate that the vasodilatatory and cytoprotective effects of CGRP at the gastric level implicate, at least in part, NO-dependent mechanisms (b.r. No. 41). Implication of nitric oxide in the smooth muscle relaxation exercised by CGRP has also been demonstrated (b.r. No. 42).

Glycine is released during the metabolic processes of amtolmetin guacyl. Since glycine determines an increase in glomerular filtrate and this effect is inhibited by an antagonist of NO synthesis, it is legitimate to consider NO as the mediator of the action exercised on this mechanism by glycine. Hence, since NO is implicated in the mechanism of action of CGRP and of glycine, and the latter aminoacid is one of the amtolmetin guacyl metabolites, we may deduce that glycine also contributes to the positive renal effects exercised by the product.

From the results summarized above amtolmetin guacyl emerges as an NSAID with a completely new profile necessitating specific indications in use: in particular, being an anti-secretory drug and inhibitor of gastric motility, it has to be administered on an empty stomach so as not to interfere with normal digestive processes. It has to be stressed that this is the first case in which a NSAID is administered on empty stomach, while all other NSAIDs administered invariably after meals. Moreover, for what explained above, amtolmetin guacyl in order to have its gastro- and renal-protective effect has to be administered without concomitant assumption of H₁-receptor inhibiting drugs.

In this respect it has to be stressed that the above indications enclose only the teaching that in order to have its gastro- and renal-protective effect amtolmetin guacyl has to be administered under particular conditions, without giving any indications which belong to the physician's responsabilities.

In conclusion, the data presented indicate that amtolmetin guacyl is an anti-inflammatory drug with outstanding characteristics, which make its use possible in patients in whom the administration of NSAIDs is usually contraindicated.

### 4) Clinics

Once the mechanism of action of MED 15 was found and its pharmacology defined we proceeded to establish the efficacy and tolerability in man.

The pharmacodynamic profile of amtolmetin guacyl must necessarily depart from its antiphlogistic and antalgic properties, the two aspects of most interest to both the patient and the physician.

To do this we must analyze the different efficacy of amtolmetin guacyl compared with some of the most widely used NSAIDs in the treatment of the four following conditions:
1) - rheumatoid arthritis
2) - osteoarthritis
3) - extra-articular rheumatism
4) - post-operatory pain.

Following are summarized the results obtained on a total of 1596 patients in 37 clinical trials.

### Rheumatoid arthritis

Amtolmetin guacyl was compared with ibuprofen, indomethacin, piroxicam and tolmetin.

As reported in Table 11, when administered to 150 patients with rheumatoid arthritis in the active phase Amtolmetin guacyl demonstrated significantly more rapid and marked anti-inflammatory activity compared to the reference NSAIDs.

The anti-inflammatory activity of Amtolmetin guacyl in rheumatoid arthritis would make it the product of choice in the treatment of this disease.

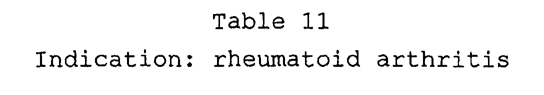

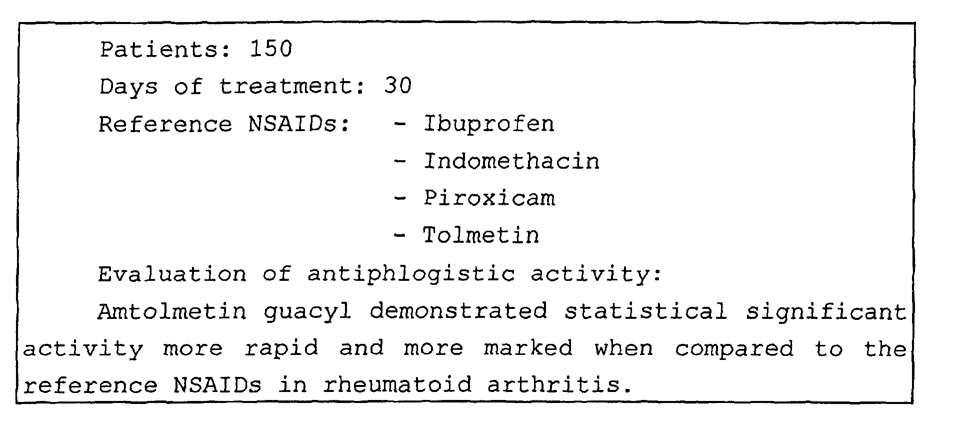

### Osteoarthritis

In this degenerative disease amtolmetin guacyl was compared with diclofenac, diflunisal, flurbiprofen, ibuprofen, ketoprofen, naproxen and tolmetin.
Amtolmetin guacyl demonstrated statistically significant more rapid and more marked activity, as reported in Table 12.

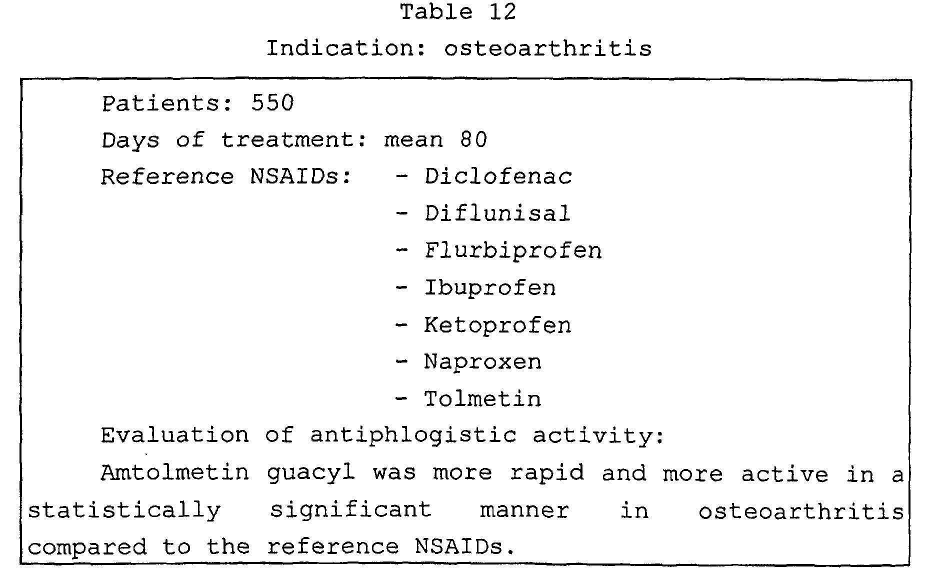

The anti-inflammatory activity of amtolmetin guacyl in osteoarthritis would make it the product of choice in the treatment of this disease.

### Extra-articular rheumatism

In extra-articular rheumatism amtolmetin guacyl was compared with diclofenac, naproxen and piperazine propionate. The results are shown in Table 13.

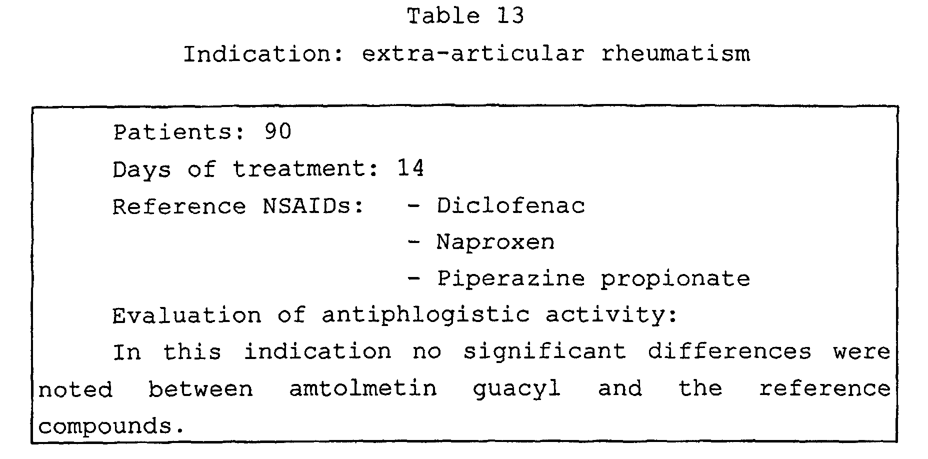

### Post-operative pain

As reported in Table 14, the analgesic properties of amtolmetin guacyl were tested in postoperative pain with good results.

The same can be said in the treatment of post-traumatic arthralgia.

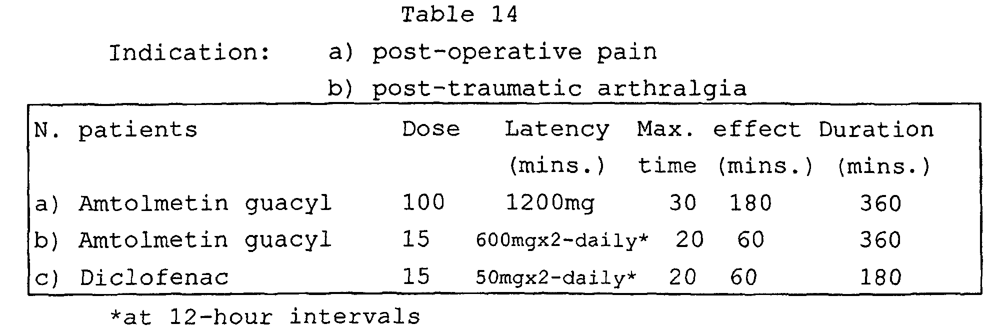

The data obtained indicate that the antalgic effect is statistically significant in post-operative pain and this effect is already evaluable at 30' following administration. The peak effect is obtained only after 2 hours, and it is maintained for 6 hours.

When compared with diclofenac in post-traumatic arthralgia Amtolmetin guacyl showed the same latency time (20 minutes), the same time to reach peak effect (1 hour), but a longer duration of effect (6 hours as opposed to 3 hours).

### CONCLUSIONS

Clinical studies demonstrate that amtolmetin guacyl has very marked anti-inflammatory activity, primarily in the treatment of rheumatoid arthritis, osteoarthritis and post-operative pain. There were no differences compared to the other NSAIDs in the treatment of extra-articular rheumatism. The results are summarised in Table 15.

The singularity of the compound lies in the long duration of the peak effect (6 hours), unlike all of the other NSAIDs.

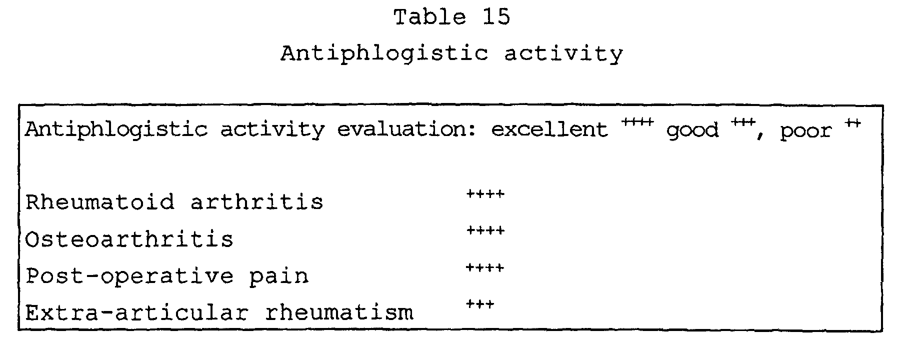

### II Parameter: tolerability

Drug tolerability is important for the correct calculation of the risk/benefit ratio. Of relevance for NSAIDs is the recognition of renal side-effects.

### A) NSAIDs and gastric damage

The incidence of the undesirable side effects of NSAIDs on the gastric mucosa varies depending on the drug structure and its pharmaceutical form. The buffered effervescent formulations, for example, avoid the gastric mucosa/compound primary damage but not damage the mucosa after re-cycling.

With the NSAIDs known to date the incidence of side effects involving the gastric mucosa is about 20-30%, the elderly, in whom the adaptive processes are often slowed down or compromised, being the most affected.

NSAID-related gastric damage is due to the inhibition of gastric prostaglandins which results in a NSAID-related hydrochloric acid hypersecretion; in fact NSAIDs by inhibiting all prostaglandin synthesis (and this is inherent to their anti-inflammatory activity), also protective prostaglandins of the gastric mucosa.

### Amtolmetin guacyl and gastric protection

Amtolmetin guacyl shows good anti-inflammatory activity in man and the pharmacological studies conducted in rats showed that this effect is due to inhibition of both COX1 (cyclooxygenase 1) and COX2 (cyclooxygenase 2).

Amtolmetin guacyl therefore also inhibits the COXl known to induce the synthesis of those prostaglandins which protect the gastric mucosa and the renal flow as well as the synthesis of TXA₂ (thromboxane A₂), a potent platelet aggregating agent. For all of these reasons the drug was expected to induce gastric damage and inhibit platelet aggregation.

Amtolmetin guacyl does indeed demonstrate significant platelet antiaggregating properties, but it has also demonstrated in clinics (with before/after endoscopies) not to induce gastric lesions, especially when administered on an empty stomach.

The mechanism of action of amtolmetin guacyl is linked to stimulation of both vanilloid receptors and CGRP (calcitonin gene-related peptide) receptors, as demonstrated using specific inhibitors. Moreover, the structure of MED 15 contains a vanilloid moiety; this appears to be the reason for its specific stimulating effect. The various chemical structures are reported in fig. 15 for comparison.

Amtolmetin guacyl per se fails to modify basal acid secretion; its gastroprotective effect arises only when H⁺ production exceeds a certain limit.

### B) Renal tolerability.

Inhibition of prostaglandin synthesis is known to reflect negatively on renal function since prostaglandins are delegated to the modulation of renal blood flow and they are physiologically released to counteract the renovasoconstriction produced by angiotensin II.

CGRP overflow from the stomach into the bloodstream produces visible effects in patients (red flushed appearance) and effects on the kidney (increase in diuresis), counteracting the effects of prostaglandin inhibition.

To shed some light on the possible exploitation of this unexpected effect on the kidney we listed the classes of patients which show the highest risk of developing renal failures when treated with NSAIDs.

**Table 16**

| Patient groups at highest risk of developing NSAID-induced renal failure | |
|---|---|
| Certain | - congestive heart failure |
| | - hepatic insufficiencies with ascites |
| | - nephrosis |
| | - disorders involving decrease of blood volume |
| | (ex. dehydration) |
| | - chronic glomerulonephritis |
| | - chronic renal failure |
| | - systemic lupos erythematosus |
| | - multiple myeloma |
| Possible | - the elderly (especially diabetics |
| | and hypertensives) |
| | - atherosclerotic-related cardiovascular |
| | disease |
| | - gouty arthritis (precipitated by diuretics |
| | in the elderly) |
| | - patients submitted to general anaesthesia |
| | - patients with sepsis, endotoxemia and serious |
| | infections |
| | - concomitant diuretic treatment |
| | - patients on low-salt diets |
| | - chronic pyelonephritis |

### Amtolmetin guacyl and renal function

Despite the fact that amtolmetin guacyl is a NSAID, it has been surprisingly found that it antagonizes the renal vasoconstriction provoked by the inhibition of prostaglandin synthesis.

As proof of the above, studies carried out in rats demonstrated that a single dose of Amtolmetin guacyl increased diuresis in these animals by an average of 10%, whilst diclofenac decreased it by an average of 10%.

Repeated doses (one per day) for 8 days increased diuresis in the treated rats by 35% (see fig. 13).

### Tolerability: conclusions

The tolerability of amtolmetin guacyl is very good and no significant variations of the routine biological parameters were observed during or after treatment.

The organs most open to damage by the NSAIDs, the gastrointestinal system and the kidneys, benefit from the protective activity of Amtolmetin guacyl, without detriment to the therapeutic activity of the drug.

At present in clinics NSAID-related side-effects are minimized by the simultaneous delivery of H₂-receptor antagonists or protonic pump inhibitors or gastroprotective prostaglandins. All these treatments have their own side-effects. In our case this protective effect is obtained with a single drug, improving therapy acceptability on the part of the patient, especially in the long-term treatments.

Confirmation of the above described advantages is the very low incidence of treatment suspension for adverse events found in clinics (Table 17), which is not the case for most of NSAIDs known in the art (Table 18).

Table 17 reports the results of amtolmetin guacyl in a cohort of 949 patients.

**Table 17**

| Suspensions of treatment for adverse events with amtolmetin guacyl | | | |
|---|---|---|---|
| Days of treatment | N. patients | Treatment | suspended % |
| 10 | 190 | 3 | 1,6 |
| 21 | 70 | 0 | |
| 30 | 140 | 1 | 0,7 |
| 60 | 225 | 0 | |
| 90 | 225 | 0 | |
| 120 | 25 | 0 | |
| 150 | 49 | 0 | |
| 180 | 25 | 0 | |
| Total | 949 | 4 | 0,4 |

| Incidence of treatment suspension for adverse events: meloxicam and diclofenac | | |
|---|---|---|
| | Meloxicam | Diclofenac 100mg |
| Days of treatment | 180 | 180 |
| N. patients | 336 | 336 |
| Suspensions of treatment | 42 | 63 |
| % of totale | 12,4 | 18,7 |

As can be seen from this table, the differences of tolerability between Amtolmetin guacyl, meloxicam and diclofenac appear wide; it must be taken into consideration that the 6-month (a total of 60.480 days) duration of the meloxicam and diclofenac studies well compares with the duration of the Amtolmetin guacyl studies (a total of 56.170 days)

In any case all reports of gastric discomfort (4%) were of a minor nature resolved spontaneously during the course of the treatment. These minor events may be explained by the possibility that the drug was taken after meals or in concomitance with antihistamine (anti-H₁) agents, which inhibit the gastroprotective effect of amtolmetin guacyl whilst maintaining its antiinflammatory effect. Consequently amtolmetin guacyl, to show its activity, has to be administered on empty stomach and without a concomitant administration of H₁-receptor inhibiting drug.

In summary, due to its antiinflammatory properties and protective effect on the stomach and the kidney, the following patient categories are specifically good responders to MED 15 treatment:
subjects with a need for a long term treatment with a NSAID, subjects with specific gastric and/or renal intolerance to NSAID's, subjects with preexisting gastritis and/or gastric and/or gastroduodenal lesions, subjects with NSAID-related renal impairment due to
vasoconstriction, subjects with reduced renal function or in damage thereof.

### BIBLIOGRAPHY

1) Matera Prof M., 1st. di Farmacoiogia, Cattedra di Chemioterapia, Facolta di Medicina e Chirurgia, Università di Catania, 1986, "Evaluation of the pharmacodynamic activity of MED15 - antinflammatory, analgesic and antipyretic activity".
2) Seed M.P., Greenslade K.J, Willoughby D.A., William Harvey Research Inst., London, 1996, "Study into the Effects of MED15 on Monoarticular Arthritis in the Rat"
3) Greenslade K.J., Moore A.R., Willoughby D.A., William Harvey Research Inst., London, 1996, "Study into the Effects of MED15 on Carrageenan Pleurisy in the Rat".
4) Matera Prof. M., Ist. di Farmacologia, Cattedra di Chemioterapia, Facolta di Medicina e Chirurgia, Università di Catania, 1989, "Effects of MED15 on Blood Pressure, ECG and Heartbeat Frequency in the Anaesthetized Rat"
5) Matera Prof. M., 1st. di Farmacologia, Cattedra di Chemioterapia, Facoltà di Medicina e Chirurgia, Universita di Catania, 1989, "Study of the Acute Toxicity of MED15".
6) Zühlke U., Hazleton Research Labs., 1992, Project 419-500, "Medosan15 - 52-week oral (gavage) chronic toxicity study in the rat with an eight-week treatment-free period".
7) Zühlke U., Hazleton Research Labs, Project 419-501, 1992, "Medosan15 - 52-week oral (gavage) chronic toxicity study in the cynomolgous monkey with an eight-week treatment-free period".
8) Allen P.A., Mladenovic P., Terrier Ch., RCC Research % Consulting Company, 1989, "Embryotoxicity Study (Including Teratogenicity) with MED15 in the Rat and in the Rat".
9) Allen P.A., Mladenovic P., Terrier Ch., RCC Research % Consulting Company, 1989, "Embryotoxicity Study (Including Teratogenicity) with MED15 in the Rat and in the Rabbit".
10) Allen P.A., Mladenovic P., RCC Research & Consulting Company, 1989, "Fertility and General Reproduction Study with MED15 in the Rat".
11) Forster R., Monaco M., Nunziata A., LSR-RTC, 1983, "Reverse Mutation in Salmonella Typhimurium. Test substance: Medosan15 (Ames Test)"
12) Forster R., Monaco M., Nunziata A., LSR-RTC, 1983, "Forward Mutation in Schizosaccharomyces Pombe 1. Test substance. Medosan15"
13) Forster R., Monaco M. Nunziata A., LSR-RTC, 1983, "Mitotic Gene Conversion in Saccharomyces Cerevisiae D4 Test substance Medosan15"
14) Conz A., Fumero S., RBM Antione Marxer, 1989, "Chromosome Aberrations in Human Lymphocytes Cultured *in vitro*. Test substance: MED15".
15) Conz A., Fumero S., RBM Antione Marxer, 1989, "Micronucleus Test in Rat Bone Marrow. Test substance: MED15".
16) Germano O., Maraschin R., RBM Antione Marxer, 1995, "Evaluation of the carcinogenicity of the test article ST679/MED15 administered by oral route to the rat for a period covering most of its lifespan".
17) Tubaro E., Belogi L., Mezzadri C.M., Ruco L., Stoppacciaro A. "Studies on the gastric tolerability of the new non-steroidal anti-inflammatory drug amtolmetin guacyl". Arzneim Forsch Drug Res 1995, 45:1298-1302.
18) Bunce K.T., Parsons M E. "A quantitative study of metiamide, a histamine H₂-antagonist, on the isolated whole rat stomach" J Physiol 1976;258:453-465,"
19) Boughton-Smith N.K., Whittle B.J R. "The gastric antisecretory actions of prostaglandin E₂ and stable prostacyclin analogues against different secretagogues in perfused whole stomachs of rat of mouse *in vitro*" Br J Pharmacol 1981;72:291-298.
20) Wan B.Y.C. "Metiamide and stimulated acid secretion from the isolated non-distended and distended mouse stomach" J Physiol 1977;266:327-346.
21) Suzuki A., Kamejama J., Tsukamoto M., Suzuki Y. "Bicarbonate secretion in isolated guinea-pig antrum" J Clin Gastroenterol, 1990;12(Suppl):14-18.
22) Kameyama J., Suzuki A., Tsukamoto M., Suzuki Y., Kaneko K. "Effects of bile acids and bilirubin on bicarbonate secretion of isolated guinea-pig antrum" J Clin Gastroenterol, 1992;14(Suppl):102-106.
23) Leithold M., Fleissig W., Merk A. "Anti-ulcer and secretion-inhibitory properties of the tricyclic derivative Doxepin in rats and dogs". Arzneim Forsch Drug Res 1984;34:468-473.
24) Munt P.I., Williams C. Huntingdon Life Sciences U.K., 1996 "MED15 (amtolmetine guacyl) - Effect on gastric acid secretion in the anaesthetized rat".
25) Munt P.I., Williams C. Huntingdon Life Sciences U.K., 1996 "MED15 (amtolmetine guacyl) - Effect on gastric acid secretion *in vitro*".
26) Bertaccini G., Coruzzi G, Scarpignato C. "Effects of alkyl analogues of histamine and metiamide on the isolated guinea pig heart" Pharmacology 1931,22 101-107
27) Odes H.S., Hogan D.L., Steinbach H.H., Ballesteros M.A., Koss M.A., Isenberg J.I. "Measurement of gastric bicarbonate secretion in the human stomach: different methods produce discordant results". Scand J Gastroenterol 1992,27.829-836
28) Segawa K., Arisawa T., Niwa Y, Kato T., Tsukamoto Y., Goto H., Hayakawa T., Nakazawa S. "The relationship between titrated acidity (mEq/l) and pH of human gastric juice: a study based on the data estimated by pHmeter". Nippon Shokakibyo Gakkai Zasshi 1994;91:849-853.
29) Barker L.A. "Histamine H1 and muscarinic receptor antagonist activity of cimetidine and tiotidine in the guinea pig isolated ileum". Agents Actions 1981;11:699-705
30) Vane J. "A sensitive method for the assay of 5H-hydroxytryptamine". Br J Pharmac 1957;12:344-349.
31) Manela F.D., Ren J., Gao J., McGuigan J.E., Harty R F. "Calcitonin gene-related peptide modulates acid-mediated reguation of somatostatin and gastrin release from rat antrum" Gastroenterology 1995;109:701-706.
32) Ren J., Young R.L., Lassiter D.C., Harty R.F. "Calcitonin gene-related peptide mediates capsaicin-induced neuroendocrine responses in rat antrum". Gastroenterology 1993;104:485-491.
33) Takeuchi K., Ohuchi T., Matsumoto J., Okabe S. "Regulation of gastroduodenal bicarbonate secretion by capsaicin-sensitive sensory neurons in rats". J Clin Gastroenteral 1993,17(Suppl):533-539.
34) Yeh J.L., Lo Y.C., Wang Y., Chen I.J. "Cardiovascular interactions of nonivamide, glyceryl nonivamide, capsaicin analogues, and substance P antagonist in rats". Brain Res Bull 1993;30:641-648.
35) Wu P.C., Fang *J.Y.,* Huang Y.B., Tsai Y.H. "In vitro effect of penetration enhancers on sodium nonivamide acetate in rat skin". Biol Pharm Bull 1995:18:1790-1792,
36) WU J.R., Fann S.F., Yeh J.L., Lo Y.C., Huang T.Y., Chen I.J. "Multiple sensory and functional effects of non-phenolic aminodimethylene nonivamide: an approach to capsaicin antagonist". Gen Pharmacol 1996;27;151-158.
37) Mathison R., Davison J.S. "Regulation of jejunal arterioles by capsaicin-sensitive nerves in Nippostrongylus brasiliensis-sensitized rats". J Pharmacol Exp Ther 1995;273:337-343.
38) Wallace J.L., McKnight G.W., Befus A.D. "Capsaicin-induced hyperemia in the stomach: possible contribution of mast cells". Am J Physiol 1992;263:G209-14.
39) Palla R., Parrini M., Panichi V., Andreini B., De Pietro S. "Acute effects of calcitonin gene related peptide on renal haemodynamics and renin and angiotensin II secretion in patients with renal disease". Int J Tissue React 1995;17:43-49.
40) Tache Y., Raybould Y., Wei J.Y. "Central and peripheral actions of calcitonin gene related peptide on gastric secretory and motor function" Adv Exp Med Biol 1991;298.183-198.
41) Lamrecht N., Burchert M., Respondek M., Muller K.M., Peskar B.M. "Role of CGRP and NO in the gastroprotective effect of capsaicin in the rat. Gastroenterology 1993;104:1371-1380
42) Kline L.W., Pang P.K.T. "Nitric oxide modulates the CGRP-induced relaxation in guinea pig gallbladder strips *in vitro*". Rag Pept 1994;50.207-212.
43) Garcia G.E., Hammond T.C., Wead L.M., Mendonca M.M., Brown N.R., Gabbai F.B. "Effect of angiotensin II on the renal response to aminoacid in rats" Am J Kidney Dis 1996; I.115-23.

## Claims

1. Use of the compound 2-methoxyphenyl-1-methyl-5p-methylbenzoyl-pyrrol-2-acetamido acetate for the manufacture of a drug for counteracting NSAID-related renal vasoconstriction.

2. Use according to claim 1, for the manufacture of a drug for treatment of one or more than one of the diseases belonging to the class formed by congestive heart failure, nephrosis, chronic glomerulonephritis, chronic renal failure.

3. Use according to claim 1, wherein said NSAID-related renal vasoconstriction may intervene in one or more than one of the diseases belonging to the class formed by atherosclerotic-related cardiovascular diseases, gouty arthritis, chronic pyelonephritis, diseases related to general anaesthesia, diseases related to sepsis, endotoxemia and infections, diseases requiring a diuretic treatment and, low-salt diets, diabetes, hypertension.

4. Use according to any one of the preceding claims, wherein the compound 2-methoxyphenyl-1-methyl-5p-methylbenzoyl-pyrrol-2-acetamido acetate is administered on empty stomach and without a simultaneous assumption of H₁-receptors inhibiting drugs.

## Patentansprüche

1. Verwendung der Verbindung 2-Methoxyphenyl-1-methyl-5p-methylbenzoyl-pyrrol-2-acetamidoacetat zum Herstellen eines Arzneimittels zum Entgegenwirken einer mit NSAID im Zusammenhang stehenden renalen Vasokonstriktion.

2. Verwendung nach Anspruch 1 zum Herstellen eines Arzneimittels für die Behandlung von einer oder mehr als einer der Erkrankungen, die zu der Klasse gehören, die durch dekompensierte Herzinsuffizienz, Nephrose, chronische Glomerulonephritis und chronisches Nierenversagen gebildet wird.

3. Verwendung nach Anspruch 1, wobei die mit NSAID im Zusammenhang stehende renale Vasokonstriktion bei einer oder mehr als einer der Erkrankungen auftreten kann, die zu der Klasse gehören, die durch mit Atherosklerose im Zusammenhang stehende Herz-Kreislauf-Erkrankungen, Gichtarthritis, chronische Nierenbeckenentzündung, Erkrankungen, die mit einer Allgemeinanästhesie im Zusammenhang stehen, Erkrankungen, die mit einer Sepsis, Endotoxämie und Infektionen im Zusammenhang stehen, Erkrankungen, die eine diuretische Behandlung und salzarme Diäten erfordern, Diabetes und Bluthochdruck gebildet wird.

4. Verwendung nach einem der vorangehenden Ansprüche, wobei die Verbindung 2-Methoxyphenyl-1-methyl-5p-methylbenzoyl-pyrrol-2-acetamidoacetat in den leeren Magen und ohne gleichzeitige Anwendung von H₁-Rezeptoren hemmenden Arzneimitteln verabreicht wird.

## Revendications

1. Utilisation du composé 2-méthoxyphényl-1-méthyl-5p-méthylbenzoyl-pyrrol-2-acétamido acétate pour la fabrication d'un médicament destiné à contrecarrer la vasoconstriction rénale associée aux médicaments anti-inflammatoires non stéroïdiens.

2. Utilisation selon la revendication 1, dans laquelle ladite vasoconstriction rénale associée aux médicaments anti-inflammatoires non stéroïdiens peut intervenir dans une ou plusieurs des maladies appartenant au groupe comprenant les insuffisances cardiaques congestives, la néphrose, la glomérulonéphrite chronique, les insuffisances rénales chroniques.

3. Utilisation selon la revendication 1, dans laquelle ladite vasoconstriction rénale associée aux médicaments anti-inflammatoires non stéroïdiens peut intervenir dans une ou plusieurs des maladies appartenant au groupe comprenant les maladies cardio-vasculaires associées à l'artériosclérose, l'arthrite goutteuse, la pyélonéphrite chronique, les maladies associées à l'anesthésie générale, les maladies associées à la sepsie, l'endotoxémie et les infections, les maladies nécessitant un traitement diurétique et les régimes sans sel, les diabètes, l'hypertension.

4. Utilisation selon l'une quelconque des revendications précédentes, dans laquelle le composé 2-méthoxyphényl-1-méthyl-5p-méthylbenzoyl-pyrrol-2-acétamido acétate est administré dans l'estomac vide et sans une prise simultanée de médicaments inhibant les récepteurs H₁.
